# EUROPÄISCHE PATENTSCHRIFT

(11) **EP 2 387 451 B1**
(45) Veröffentlichungstag und Bekanntmachung des Hinweises auf die Patenterteilung: **16.03.2016**
(21) Anmeldenummer: 10701613.1
(22) Anmeldetag: 05.01.2010
(51) Int. Cl.: B01D 53/86, B01J 8/02, B01J 23/38, C01B 21/22, A61M 16/00

(54) **VORRICHTUNG UND VERFAHREN ZUR ZERSETZUNG VON LACHGAS IN EINEM ADIABATEN FESTBETTREAKTOR**
APPARATUS AND PROCESS FOR DECOMPOSING DINITROGEN MONOXIDE IN AN ADIABATIC FIXED BED REACTOR
DISPOSITIF ET PROCÉDÉ DE DÉCOMPOSITION DE GAZ HILARANT DANS UN RÉACTEUR ADIABATIQUE À LIT FIXE

(30) Priorität: 13.01.2009 DE 102009004431
(43) Veröffentlichungstag der Anmeldung: 23.11.2011
(73) Patentinhaber: Linde AG, 80331 München (DE)
(72) Erfinder: BERAN, Franz, 81476 München (DE); HOFMANN, Karl-Heinz, 82110 Germering (DE); SCHÖDEL, Nicole, 81477 München (DE); SCHMEHL, Wolfgang, 22525 Hamburg (DE); WENNING, Ulrike, 82049 Pullach (DE); ZANDER, Hans-Jörg, 81479 München (DE)
(74) Vertreter: Dörries, Hans Ulrich
(86) Internationale Anmeldenummer: PCT/EP2010/000013
(87) Internationale Veröffentlichungsnummer: WO 2010/081642

(56) Entgegenhaltungen:
- EP-A1- 2 165 756
- WO-A1-2006/059506
- WO-A2-02/26355
- JP-A- 55 031 463
- US-A- 3 467 492

## Beschreibung

Die Erfindung betrifft eine Vorrichtung und ein Verfahren zur Zersetzung von Lachgas (N₂O) in einem Festbettreaktor. Eine derartige Vorrichtung und ein entsprechendes Verfahren sind beispielsweise aus der US 7235222 B2, der US 2006/0008401 A1 oder der WO 2006/059506 A1 bekannt.

Distickstoffoxid oder Lachgas ist ein ungiftiges farbloses Gas, das seit langem als Anästhesiemittel oder Analgesiemittel in Kombination mit anderen Werkstoffen eingesetzt wird. Darüber hinaus entsteht Lachgas auch bei der Salpetersäureherstellung als Nebenprodukt oder wird in organischen Synthesen eingesetzt. Bei der Verwendung als Anästhesie- bzw. Analgesiemittel besteht ein Problem darin, dass das vom Patienten ausgeatmete Lachgas sich im Behandlungsraum über die bestehenden maximal zulässigen Arbeitsplatzkonzentrationen aufkonzentrieren kann und somit auch auf längere Zeit hinweg zu einer übermäßigen Belastung des medizinischen Personals führen kann.

Das zunehmende Umweltbewusstsein hat darüber hinaus die Aufmerksamkeit darauf gelenkt, dass Lachgas auch beachtlich zum Treibhauseffekt und zur Zerstörung der Ozonschicht beiträgt.

Es besteht daher die Nachfrage nach einer Vorrichtung und einem Verfahren zur effektiven Zersetzung von Lachgas aus verschiedenen Quellen, sei es aus medizinischen Behandlungen, aus der Salpetersäureproduktion, aus Abgasen verschiedener organischer Synthesen, bei der Reinigung/Entleerung von Gasflaschen oder aus Abgasströmen bei der Lachgasherstellung selbst, so dass kein Lachgas in die Umgebung freigesetzt wird.

Die oben genannten Patente und Patentanmeldungen befassen sich mit der Aufarbeitung von Lachgas aus medizinischen Anwendungen und schlagen im Wesentlichen vor, das Lachgas, das in mehreren Behandlung- bzw. Operationsräumen eines Klinikums anfällt, zu sammeln und einer gemeinsamen katalytischen Aufbereitung zu zuführen. Hierfür werden vorzugsweise Katalysatoren eingesetzt, die eine Lachgaszersetzung bei relativ niedrigen Temperaturen, das heißt unter 600°C, ermöglichen. Verschiedene Adsorber können der katalytischen Aufbereitung vorgeschaltet sein, um Störkomponenten, wie Sevofluran, Desfluran, Isofluran, Halothan oder andere halogenierte Kohlenwasserstoffe abzuscheiden.

Die Erfinder der vorliegenden Anmeldung haben darüber hinaus in der Europäischen Patentanmeldung EP 08164753.9, die zum Anmeldezeitpunkt der vorliegenden Anmeldung noch nicht veröffentlicht ist, selbst eine Vorrichtung und ein Verfahren zur Zersetzung von Lachgas vorgeschlagen, das vorsieht, das bei einem einzelnen Patienten anfallende Lachgas direkt vor Ort im Behandlungszimmer umzusetzen, was ein aufwändiges Sammeln verschiedener Lachgasströme in dem Klinikum erübrigt.

Andere gegenwärtig kommerziell erhältliche Verfahren zur Lachgasentfernung basieren meist auf einer überwiegend thermischen Zersetzung von Lachgas bei über 800°C.

Den moderneren katalytischen Verfahren, die bei Temperaturen unterhalb von 600°C arbeiten, ist ein Problem gemeinsam, nämlich dass die Zersetzung von Lachgas stark exotherm ist. Das heißt, würde das vom Patienten ausgeatmete Gas ohne weitere Vorbehandlung einer katalytischen Zersetzung unterworfen, so würde sich auf Grund der hohen Lachgaskonzentration von bis zu 70% eine derartig starke Exothermie bzw. Hitzentwicklung am Katalysator zeigen, dass dieser mit an Sicherheit grenzender Wahrscheinlichkeit irreversibel geschädigt würde. Die gängigen Lachgasentfernungsverfahren, wie sie vorangehend geschildert wurden, haben daher die Gemeinsamkeit, dass der Gasstrom auf eine Lachgaskonzentration von < 5%, mit einem anderen lachgasfreien Gas verdünnt wird. Darüber hinaus sind Maßnahmen vorgesehen, um den Katalysator entsprechend isotherm betreiben zu können, das heißt diesen zu kühlen bzw. zu erwärmen.

US 6,056,928 bezieht sich auf ein Verfahren zum Entfernen von Stickoxiden aus einem Gasfluss.

WO2006/059506 A1 bezieht sich auf ein Behandlungsverfahren und eine Behandlungsvorrichtung für Gas, welches Stickoxide umfasst.

Angesichts dieses Stands der Technik ist es eine Aufgabe der Erfindung, eine verbesserte Vorrichtung und ein verbessertes Verfahren zur Zersetzung von Lachgas bereitzustellen, das ohne hohen Energieaufwand oder teure, anspruchvolle Werkstoffe, insbesondere temperaturbeständige Werkstoffe, einen sicheren Anlagenbetrieb ermöglicht.

Gelöst wird die obige Aufgabe durch eine Vorrichtung nach Anspruch 1 und ein Verfahren nach Anspruch 10. Die abhängigen Ansprüche beziehen sich auf weitere vorteilhafte Ausgestaltungen der Erfindung.

Die Erfindung wird aus der folgenden Beschreibung unter Bezugnahme auf die beiliegende Zeichnung verdeutlicht.

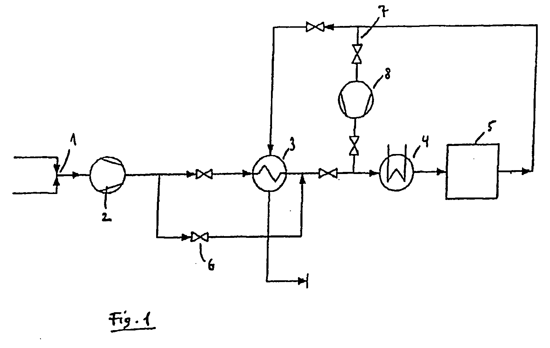

Figur 1 zeigt ein Diagramm einer beispielhaften Ausführungsform einer Vorrichtung zur Zersetzung von Lachgas.

Wie in Figur 1 zu sehen ist, umfasst die erfindungsgemäße Vorrichtung einen Gaseinlass 1, z.B. eine Atemmaske, durch die das lachgashaltige Gas eintritt. Bezugsziffer 2 bezeichnet ein Gebläse, um das Eintrittsgas über die nachfolgenden Bauteile zu fördern. Dieses Gas wird durch ein Wärmetauschermittel bzw. Wärmetauscher 3 geleitet und gelangt in eine Heizvorrichtung 4. Das die Heizvorrichtung anschließend wieder verlassende Gas wird dem eigentlichen Reaktor 5 zugeführt. Der Reaktor 5, nämlich ein Festbettreaktor, ist mit einem geeigneten Katalysator gefüllt, wobei zur Zeit ein Edelmetallkatalysator, insbesondre Palladium oder Rhodium, auf einem geeigneten Träger bevorzugt ist. Hinsichtlich der konkreten Ausgestaltung des Katalysators unterliegt die vorliegende Erfindung keiner besonderen Beschränkung. Es ist auch möglich, eine Katalysatoranordnung zu verwenden, wie sie beispielsweise in der WO 2006/009453 A1 gezeigt ist.

Das den Reaktor verlassende, im Wesentlichen lachgasfreie Gas, wird über eine Gasleitung zum Wärmetauscher 3 geführt, wo es die durch die Exothermie der Lachgaszersetzung bzw. durch das Heizelement 4 aufgenommene Wärme an das lachgashaltige Eintrittsgas abgibt. Wahlweise können noch zusätzliche Kühler vorgesehen sein, so dass das Abgas mit einer Temperatur von ca. 40°C oder weniger in die Umgebung abgegeben wird.

Erfindungsgemäß kann darüber hinaus auch ein Rückführmittel 7, 8, etwa mit einem Ventil und einem Gebläse oder Verdichter, vorgesehen sein, mit dem es möglich ist, das den Katalysator verlassende Gas in den lachgashaltigen Eintrittsgasstrom - vor oder nach dem Heizelement - zu mischen und so zurück zu führen, um die Lachgaskonzentration im Eintrittgasstrom abzusenken und das Eintrittsgas zu erwärmen.

Erfindungsgemäß ist darüber hinaus noch ein Bypass 6 vorgesehen, mit dem es möglich ist, das Eintrittsgas ganz oder teilweise am Wärmetauscher 3 vorbei zu führen. Dies ist insbesondere dann hilfreich, wenn auf Grund der Exothermie der Lachgaszersetzungsreaktion ein zu starker Temperaturanstieg zu befürchten ist.

Mit einem weiteren nicht gezeigten Bypass kann es ermöglicht werden, das lachgashaltige Gas auch an der Heizvorrichtung ganz oder teilweise vorbei zu führen.

Als ein mögliches Ausführungsbeispiel der Erfindung wird im Folgenden der Einsatz der erfindungsgemäßen Vorrichtung im Zusammenhang mit der Zersetzung vom Lachgas im Atemgas eines Patienten betrachtet. Hierbei treten besondere Schwierigkeiten auf, da der ausgeatmete Gasstrom bis zu 50% - 70% Lachgas enthält, wobei der Rest hauptsächlich Sauerstoff ist. Darüber hinaus sind kleinere Mengen CO₂ in dem Gas. Eine weitere Schwierigkeit ergibt sich daraus, dass das Atemgas und das zur Verdünnung beigemischte lachgasfreie Gas, meist Umgebungsluft, je nach Anwendungsfall feucht sind. Die derzeitig bevorzugten Katalysatoren zeigen eine starke Abhängigkeit der Lachgaszersetzungstemperatur von der Feuchtigkeit des Gases, wobei die Lachgaszersetzungstemperatur im feuchten Gas um 50° C oder mehr, zum Teil auch bis zu 200° C, über jener im trockenen Gas liegt.

Bedingt durch die Atmung fällt das Gas diskontinuierlich an, je nach dem ob der Patient gerade ausatmet oder nicht. Auch ist zu beachten, dass bei einigen Behandlungsmethoden der Lachgasgehalt des dem Patienten zugeführten Anästhesiegases Zeit abhängig stark variiert, so dass auch in dem ausgeatmeten Gas plötzliche Schwankungen zwischen 0% und 70% auftreten können.

Die erfindungsgemäße Vorrichtung ist konzipiert, um als mobile und kompakte Einheit direkt im oder angrenzend an den Behandlungsraum eingesetzt zu werden, wobei insbesondere auch angedacht ist, die erfindungsgemäße Vorrichtung in einem Krankenwagen zu verwenden. Dies erfordert, dass außer einer Stromversorgung die erfindungsgemäße Vorrichtung autark sein sollte, das heißt, dass sie weder einen Wasseranschluss noch die Versorgung mit anderen Prozessströmen (speziellen Verdünnungsgasen oder Reduktionsmitteln) aufweist.

Um das medizinische Personal und andere Personen zu schützten, ist es zwingend erforderlich, dass die Lachgaszersetzungsvorrichtung von außen keine zu hohe Temperatur annimmt, so dass eine Berührung nicht zu Verbrennungen führt. Die Vorrichtung ist daher thermisch so zu isolieren, dass die Außentemperaturen nicht über 40°C ansteigt.

Aus ähnlichen Gründen ist auch darauf zu achten, dass das letztendlich abgegebene Abgas diesen Grenzwert der Temperatur nach Möglichkeit nicht übersteigt.

Für die Praxis ist es darüber hinaus erforderlich, dass die Vorrichtung ohne kompliziert zu bedienende Mess- und Steuervorrichtungen auskommt. Sie sollte für den Einsatz in Krankenhäusern, auch durch weniger geschultes Personal oder in Stresssituation geeignet sein, und eine fehlerhafte Bedienung der Vorrichtung sollte weitgehend ausgeschlossen sein.

Für den Einsatz im Zusammenhang mit medizinischen Gasen gibt es hinsichtlich des verwendeten Katalysators keine besonderen Einschränkungen. Prinzipiell haben sich für den Lachgasabbau Edelmetallkatalysatoren auf einem Trägermaterial als geeignet erwiesen. Zu den geeigneten Edelmetallen zählen zum Beispiel Palladium, Rhodium, Platin, Ruthenium u.a.. Insbesondere haben sich Palladium und Rhodium als geeignet erwiesen. Für die Träger kommen Aluminiumoxide, Siliziumoxide, und Zeolite bzw. deren Mischungen und andere in Betracht.

An dem Katalysator reagiert das Lachgas zu N₂ und O₂ ab. Nebenreaktionen zu anderen Stickoxiden können vermieden werden. Bei einem trockenen Gas setzt bei einigen Katalysatorsystemen die Lachgaszersetzung bereits bei unter 200°C ein, und steigt mit der Feuchtigkeit im Gas bis auf 400°C oder 450° an. Bei den bevorzugten Katalysatorsystemen wurde bereits bei etwas über 150° C im trockenen Gas ein signifikanter Lachgasabbau beobachtet.

Bei der oben beschriebenen Anwendung in einem Krankenhaus wird die Vorrichtung im einfachsten Fall so betrieben, dass das ausgeatmete Gas des Patienten unter Berücksichtung der theoretisch möglichen Maximalkonzentration verdünnt wird, so dass sich eine maximale Lachgaskonzentration von <10%, vorzugsweise < 5%, und insbesondere < 2% einstellt. Als Gas für die Verdünnung kann jedes lachgasfreie Gas, etwa Umgebungsluft oder auch das Abgas des Katalysators selbst, verwendet werden.

Bei einer bevorzugten Ausführungsform wird ein kurzzeitiges Überschreiten der oben beschriebenen maximalen Eintrittskonzentration von 5% bzw. 2% erlaubt, hierfür ist es allerdings erforderlich, dass die Temperatur, entweder im Reaktor oder des Abgases entsprechend überwacht wird, so dass bei Überschreiten einer kritischen Temperatur Tₘₐₓ das Eintrittgas schnell weiter verdünnt werden kann, um ein Durchgehen des Reaktors oder eine Schädigung des Systems durch einen zu großen Temperaturanstieg zu vermeiden.

Aus Sicherheitsgründen ist der klinischen Anwendung bevorzugt läuft die Reaktion bei Atmosphärendruck ab, u.a. um die Atmung des Patienten nicht nachteilhaft zu beeinflussen. So lässt sich auch der Einsatz eines Kompressors umgehen, der die Kosten und die Lärmbelastung deutlich erhöhen würde. Um dennoch das Gas sicher über den Reaktor leiten zu können, ist an geeigneter Stelle ein Gebläse vorgesehene.

Das erfindungsgemäße Verfahren sieht vor, zunächst das zu behandelnde lachgashaltige Gas mit dem Heizelement auf eine Temperatur zu erwärmen, bei der die Reaktion am Katalysator einsetzt. Für den beschriebenen Fall des Atemgases eines Patienten liegt diese Temperatur bei den derzeit bevorzugten Edelmetallkatalysatoren bei ca. 300° - 400°C oder leicht darunter. Bei einem trockenen Gas, wie es beispielsweise bei der Aufarbeitung von Restgasen bei der Flaschenabfüllung auftritt, oder das durch geeignete Trocknung des Atemgases und des Verdünnungsgases erhalten werden kann, kann eine Starttemperatur von unter 300°C, vorzugsweise bei 200°C gewählt werden. Die genaue Temperatur, bei der die Lachgaszersetzung im nennenswerten Umfang beginnt, hängt von dem gewählten Katalysatorsystem ab, und ist in Abhängigkeit von diesem im Voraus zu bestimmen. Wie bekannt hängt auch der tatsächliche Umsetzungsgrad von der Temperatur ab.

Nach einer kurzen Anlaufphase, in der das lachgashaltige Gas vor dem Reaktor erwärmt wird, sorgt die Exothermie der Lachgaszersetzungsreaktion dafür, dass in dem Reaktor eine beachtliche Wärmemenge freigesetzt wird. Berechnungen zeigen, dass die Zersetzung von 0,5% Lachgas ausreicht, um das Gas um ca. 13° C zu erwärmen. Die Reaktion in dem Reaktor wird adiabat geführt, das heißt, dass der Reaktor gegenüber der Umgebung thermisch gut eingekapselt ist, so dass er sich schnell selbst erwärmt und dann ohne Zufuhr weiterer Wärmeenergie betrieben werden kann.

Das Abgas des Reaktors, das im Wesentlichen lachgasfrei ist, wird über eine Abgasleitung zu dem Wärmetauscher geführt, wo es seine Wärme möglichst vollständig an das lachgashaltige Gas vor dem Eintritt in den Reaktor, besser noch vor dem Eintritt in das Heizelement abgibt. Wenn das lachgashaltige Gas durch ein im Wesentlichen lachgasfreies Gas verdünnt wird, kann für diese Verdünnung auch das heiße Abgas des Reaktors verwendet werden, was die Ausgestaltung des Wärmetauschers vereinfacht. Dieser Ansatz erlaubt es auch bei der Verwendung im Klinikum, das System mit einem relativ trockenen Gas zu betreiben, da immer nur die im Atemgas des Patienten enthaltene Feuchtigkeit entfernt werden muss, was mit einem Adsorber, etwa einem Molsieb oder durch Blaugel leicht möglich ist.

Nach kurzer Zeit kann das Heizelement ausgeschaltet werden, und die Reaktion läuft stabil nur aufgrund der Exothermie der Lachgaszersetzung an dem Katalysator. Eine zu starke Temperaturerhöhung wird durch geeignete Verdünnung des lachgashaltigen Gases verhindert.

Bei einer bevorzugten Ausführungsform des erfindungsgemäßen Verfahrens wird in einer ersten Phase das lachgashaltige Gas, ohne Verdünnung, über das Heizelement geleitet, sodass nur eine kleine Gasmenge aufgeheizt werden muss. Dieses Gas mit der hohen Lachgaskonzentration von bis zu 70% wird dann kurzfristig über den Katalysator geleitet, wobei sich aufgrund der starken Exothermie das Abgas und der Katalysator stark erwärmt.

In einem zweiten Schritt wird dann zunehmend das lachgashaltige Gas vor dem Reaktor verdünnt, entweder wie beschrieben, mit dem Abgas des Reaktors oder mit Umgebungsluft, und das Heizelement wird ausgeschaltet.

Mit dem oben beschriebenen Ansatz ist das An- und Abfahren der Reaktion wesentlich schneller als bei bekannten Prozessen, die eine thermische Lachgaszersetzung bei 800°C vorsehen. Dies ist besonders dann von Vorteil, wenn man bedenkt, dass die erfindungsgemäße Vorrichtung in einem Behandlungsraum eingesetzt wird, indem zwischen den einzelnen Behandlungen auch längere Pausen auftreten können, in denen kein Lachgas anfällt. Andererseits ist es gerade in diesen Situation zwingend erforderlich, dass, beispielsweise bei Noteingriffen, die Lachgaszersetzungsvorrichtung sehr schnell einsatzbereit ist, und eine lange Aufwärmphase, wie sie bei herkömmlichen Vorrichtungen erforderlich wäre, ist nicht praktikabel.

Neben den oben geschilderten Verwendungen auf dem Gebiet der Medizin kann die erfindungsgemäße Vorrichtung auch in anderen Einsatzgebieten verwendet werden, beispielsweise zur Zersetzung von Lachgas bei der Gasflaschenabfüllung, wenn partiell gelehrte Flaschen aufgearbeitet werden müssen, und das in den Flaschen noch verbliebene Gas vor der Neubefüllung entsorgt werden muss. Ein anderes Einsatzgebiet ergibt sich im Zusammenhang mit den Abgasen z.B. der Salpetersäureproduktion oder Adipinsäureproduktion.

Des Weiteren kann die erfindungsgemäße Vorrichtung und das erfindungsgemäße Verfahren auch bei den gängigen Verfahren zur Herstellung von Lachgas verwendet werden, bei denen regelmäßig Abgasströme mit einem noch beachtlichen Lachgasanteil, der nicht weiter aufgearbeitet werden kann, zu entsorgen sind. Um die Umwelt zu schützten ist es zweckmäßig, auch in diesen Abgasströmen das Lachgas vor der Freisetzung in die Umgebung zu zersetzen.

Bei einigen Anwendungen sind im Lachgas noch weitere Gasbestandteile, die sich zum Teil störend auf den Prozess auswirken können. Insbesondere können teilweise oder vollständig halogenierte Kohlenwasserstoffe, zum Beispiel Sevofluran, Desfluran, Isofluran, und Halothan in medizinischen Gasgemischen vorhanden sein. Diese halogenierten und teilhalogenierten Kohlenwasserstoffe zählen zu den klassischen Katalysatorgiften und würden die Edelmetallkatalysatoren irreversibel schädigen, was zu einer beachtlichen Verkürzung der Standzeit der Katalysatoren führen würde. Zur Vermeidung dieser Schädigung kann vor dem Katalysator ein entsprechender Adsorber eingebaut werden, der diese Komponenten zurückhält. Die Entfernung der halogenierten Kohlenwasserstoffe aus der Abluft ist auch zum Schutz des medizinischen Personals von großer Wichtigkeit.

In einer nicht weiter gezeigten Ausgestaltung der Erfindung, weist die Lachgaszersetzungsvorrichtung mehrere hintereinander geschaltete adiabate Festbettreaktoren auf, in denen jeweils ein geeigneter Katalysator enthalten ist, wobei zwischen den einzelnen Festbettreaktoren Kühler bzw. Wärmetauscher vorgesehen sein können. Der Vorteil dieser Anordnung besteht darin, dass eine zu starke Überhitzung der Katalysatoren vermieden werden kann.

Ein weiterer Vorteil dieser Ausgestaltung besteht darin, dass es leicht ist, die einzelnen Katalysatoren in den adiabaten Festbettreaktoren als "Patrone" auszugestalten, die ausgetauscht werden kann. Bei einem Gesamtvolumen von beispielsweise 10 Liter Katalysator ist es möglich, dieses in drei oder vier gleich große Teile aufzuteilen, und diese, 2,5 bis 3,5 Liter großen "Patronen" je nach Bedarf auszutauschen. Da davon auszugehen ist, dass der erste Festbettreaktor am stärksten belastet wird, ist es beispielsweise möglich, nach einer gewissen Verfahrensdauer, den Katalysator aus dem ersten Festbettreaktor zu entfernen, und stattdessen den Katalysator aus dem zweiten Festbettreaktor nach vorne zu verschieben. Der Katalysator aus dem dritten Festbettreaktor wird dann in den zweiten Festbettreaktor versetzt und eine neue Patrone mit einem neuen Katalysator wird in den dritten und letzten Festbettreaktor eingeführt.

In gleicher Weise kann auch der zur Entfernung von halogenierten Kohlenwasserstoffen eventuell vorgesehene Adsorber als Kartusche ausgestaltet und ausgetauscht werden.

Die Erfindung ist nicht auf diese beschriebenen Beispiele beschränkt. Die Erfindung kann überall dort eingesetzt werden, wo Lachgas zu zersetzen ist.

Zur Erreichung des adiabaten Betriebszustands des Reaktors kann dieser durch jede geeignete Maßnahme isoliert sein. Eine besonders vorteilhafte Isolierung ist die Aufnahme des Katalysators in einen doppelwandigen Reaktorbehälter mit einer Vakuum-Isolierung.

In vorteilhafter Weise ist die erfindungsgemäße Vorrichtung darüber hinaus mit einer Steuerung versehen, die in Abhängigkeit von der Temperatur im Reaktor, der Temperatur des Abgases, der Temperatur des Eintrittsgases in den Reaktor, dem Ausmaß der Verdünnung, der Lachgaskonzentration und/oder der Zeit sowohl das Heizelement, die Verdünnung als auch die eventuellen Bypässe um das Heizelement und den Wärmetauscher steuert.

Bei einer weiteren erfindungsgemäßen Ausgestaltung ist es möglich, das Eintrittsgas, vor Eintritt in das Heizelement, durch geeignete Maßnahmen, beispielsweise ein Molsieb, zu trocknen. Dies hat den Vorteil, dass die Zersetzungstemperatur am Katalysator beachtlich abgesenkt wird. Wenn ein trockenes Gas zur Verdünnung eingesetzt wird, sei es aus einer speziellen Gasversorgung oder sei es das trockene Abgas des Katalysators, kann die Trocknervorrichtung klein und kompakt ausgeführt werden. Auch membranbasierte Trocknervorrichtungen sind erfindungsgemäß einsetzbar.

Wenn zwei oder mehr parallel geschaltete Adsorber vorgesehen sind, ist es möglich, einen dieser Adsorber aktiv für die Trocknung des Eintrittgases zu verwenden, während ein anderer in den Abgasstrom geschaltet ist, so dass das warme Abgas das adsorbierte Wasser wieder hinausträgt, und den Adsorber für den nächsten Zyklus regeneriert. Eine Mehrzahl von Adsorbern ermöglicht zeitversetzte Zyklen.

Bei einer weiteren bevorzugten Ausführungsform des erfindungsgemäßen Verfahrens wird beim Start das Eintrittsgas mit einem kleinen kompakten Trockner getrocknet und über ein Heizelement geleitet, das ebenfalls klein sein kann, da beim trockenen Gas keine hohe Anspringtemperatur erforderlich ist, also ein "Kaltstart" ermöglicht wird. Nach Erreichen der regulären Betriebstemperatur des Katalysators, z.B, 400°C, kann dann der Betrieb ohne Trocknung und zusätzliche Heizung betrieben werden.

Zur weiteren Verkleinerung des Trockners bzw. des Heizelements und des Energieaufwandes ist es möglich beim Start das nur leicht oder unverdünnte Atemgas des Patienten zu verwenden, und nach Bedarf die Verdünnung erst später zuzuschalten.

Das Heizelement ist nicht auf ein elektrisches Heizelement beschränkt, obwohl dies bevorzugt ist. Alternativ kann das lachgashaltige Eintrittsgas auch durch andere Mittel, beispielsweise ein durch chemische Reaktion beheiztes Element, erwärmt werden. Bei der Verwendung in einem Krankenwagen kann auch die Abwärme des Motors für die Heizung eingesetzt werden.

## Patentansprüche

1. Vorrichtung zur Zersetzung von Lachgas mit:
einem Gaseinlass (1) zum Zuführen eines lachgashaltigen Gases;
einem ersten Wärmetauschermittel (3) zum Austausch von Wärme zwischen einem Abgas und dem lachgashaltigen Gas;
einer Heizvorrichtung (4) zum zeitweisen Heizen des lachgashaltigen Gases;
einen Festbettreaktor (5), in dem ein Katalysator aufgenommen ist, um das Lachgas in dem lachgashaltigen Gas zu zersetzen;
einem Gasauslass, durch den das den Festbettreaktor verlassende Abgas über den Wärmetauscher abgeführt werden kann; wobei
der Katalysator zur Zersetzung von Lachgas bei Temperaturen unter 800°C geeignet ist; und
wobei der Festbettreaktor (5) als Adiabatreaktor ausgestaltet ist,
**dadurch gekennzeichnet, dass**
die Vorrichtung so ausgestaltet ist, dass die Reaktion zum katalytischen Zersetzen des Lachgases bei Atmosphärendruck abläuft und ein Gebläse vorgesehen ist, um das lachgashaltige Gas über den Festbettreaktor (5) zu leiten.

2. Vorrichtung nach Anspruch 1, **dadurch gekennzeichnet, dass** ein Rückführmittel (7, 8) vorgesehen ist, um zumindest einen Teil des Abgases mit dem lachgashaltigen Gas vor dem Festbettreaktor (5) zu vermischen.

3. Vorrichtung nach Anspruch 2, **dadurch gekennzeichnet, dass** das Rückführmittel (7, 8) ausgestaltet ist, um einen Teil des Abgases mit dem lachgashaltigen Gas vor der Heizvorrichtung (4) zu vermischen.

4. Vorrichtung nach einem der Ansprüche 1 bis 3 **dadurch gekennzeichnet, dass** ein erstes Steuermittel vorgesehen ist, um den Betrieb der Heizvorrichtung (4) in Abhängigkeit von der Temperatur des lachgashaltigen Gases, der Temperatur des Festbettreaktor (5), der Temperatur des Abgases, dem Ausmaß der Abgasrückführung, der Lachgaskonzentration und/oder der Zeit zu steuern.

5. Vorrichtung nach einem der Ansprüche 1 bis 4, **dadurch gekennzeichnet, dass** ein Bypass (6) vorgesehen ist, um das Wärmetauschermittel (3) zumindest zeitweise zu umgehen.

6. Vorrichtung nach Anspruch 5, **dadurch gekennzeichnet, dass** ein zweites Steuermittel vorgesehen ist, um den Bypass (6) in Abhängigkeit von der Temperatur des lachgashaltigen Gases, der Temperatur des Festbettreaktors (5), der Temperatur des Abgases, dem Ausmaß der Abgasrückführung, der Lachgaskonzentration und/oder der Zeit zu steuern.

7. Vorrichtung nach Anspruch 1, **dadurch gekennzeichnet, dass** ein Verdünnungsmittel vorgesehen ist, um das lachgashaltige Gas mit einem anderen lachgasfreien Gas zu verdünnen.

8. Vorrichtung nach einem der Ansprüche 1 bis 7, **dadurch gekennzeichnet, dass** eine Mehrzahl adiabater Festbettreaktoren (5) in einer Reihe vorgesehen sind, wobei optional zwischen den einzelnen Festbettreaktoren (5) jeweilige Wärmetauscher oder Kühler angebracht sind.

9. Vorrichtung nach einem der vorstehenden Ansprüche, wobei der Katalysator zur Zersetzung von Lachgas bei Temperaturen von unter 450°C, bevorzugt unter 400°C, geeignet ist.

10. Verfahren zur Zersetzung von Lachgas mit:
einem Schritt zum Zuführen eines lachgashaltigen Gases;
einem Wärmetauschschritt zum Austausch von Wärme zwischen einem Abgas und dem lachgashaltigen Gas;
einem Heizschritt zum zeitweisen Heizen des lachgashaltigen Gases;
einem Reaktionsschritt zum katalytischen Zersetzen des Lachgases in dem lachgashaltigen Gas in einem Festbettreaktor (5);
einem Schritt zum Abführen des Abgases aus dem Festbettreaktor (5) über den Wärmetauscher; wobei
der Reaktionsschritt bei einer Temperatur unter 800°C beginnt und unter adiabaten Bedingungen erfolgt,
wobei
die Reaktion bei Atmosphärendruck abläuft und das lachgashaltige Gas mittels eines Gebläses über den Festbettreaktor (5) geleitet wird.

11. Verfahren nach Anspruch 10, **dadurch gekennzeichnet, dass** das lachgashaltige Gas mit zumindest einem Teil des Abgases vor dem Festbettreaktor (5) vermischt wird.

12. Verfahren nach Anspruch 11, **dadurch gekennzeichnet, dass** das Vermischen vor dem Heizschritt erfolgt.

13. Verfahren nach einem der Ansprüche 10 bis 12, **dadurch gekennzeichnet, dass** der Heizschritt in Abhängigkeit von der Temperatur des lachgashaltigen Gases, der Temperatur des Festbettreaktor (5), der Temperatur des Abgases, dem Ausmaß der Abgasrückführung, der Lachgaskonzentration und/oder der Zeit gesteuert wird.

14. Verfahren nach einem der Ansprüche 10 bis 13, **dadurch gekennzeichnet, dass** das lachgashaltige Gas zeitweise ganz oder teilweise in einem Bypass (6) an dem Wärmetauscherschritt vorbei geführt wird.

15. Verfahren nach Anspruch 14, **dadurch gekennzeichnet, dass** der Bypass (6) in Abhängigkeit von der Temperatur des lachgashaltigen Gases, der Temperatur des Festbettreaktor (5), der Temperatur des Abgases, dem Ausmaß der Abgasrückführung, der Lachgaskonzentration und/oder der Zeit gesteuert wird.

16. Verfahren nach einem der Ansprüche 10 bis 15, **dadurch gekennzeichnet, dass** das lachgashaltige Gas mit einem anderen lachgasfreien Gas verdünnt wird.

17. Verfahren nach einem der Ansprüche 10 bis 16, **dadurch gekennzeichnet, dass** der Reaktionsschritt bei einer Temperatur unter 450°C, bevorzugt unter 400°C, beginnt.

## Claims

1. An apparatus for decomposing dinitrogen monoxide, featuring:
a gas inlet (1) for supplying a gas containing dinitrogen monoxide;
a first heat exchanging means (3) for exchanging heat between a waste gas and the gas containing dinitrogen monoxide;
a heating device (4) for intermittently heating the gas containing dinitrogen monoxide;
a fixed bed reactor (5), in which a catalyst is accommodated in order to decompose the dinitrogen monoxide in the gas containing dinitrogen monoxide;
a gas outlet, through which the waste gas exiting the fixed bed reactor can be discharged via the heat exchanger; wherein
the catalyst is suitable for the decomposition of dinitrogen monoxide at temperatures below 800°C; and
wherein the fixed bed reactor (5) is realized in the form of an adiabatic reactor,
**characterized in that**
the apparatus is realized in such a way that the reaction for catalytically decomposing the dinitrogen oxide takes place at atmospheric pressure, and **in that** a fan is provided in order to convey the gas containing dinitrogen monoxide over the fixed bed reactor (5).

2. The apparatus according to claim 1, **characterized in that** a return means (7,8) is provided in order to mix at least part of the waste gas with the gas containing dinitrogen monoxide upstream of the fixed bed reactor (5).

3. The apparatus according to claim 2, **characterized in that** the return means (7,8) is designed for mixing part of the waste gas with the gas containing dinitrogen monoxide upstream of the heating device (4).

4. The apparatus according to any one of claims 1-3, **characterized in that** a first control means is provided in order to control the operation of the heating device (4) as a function of the temperature of the gas containing dinitrogen monoxide, the temperature of the fixed bed reactor (5), the temperature of the waste gas, the extent of the waste gas return, the dinitrogen monoxide concentration and/or time.

5. The apparatus according to any one of claims 1-4, **characterized in that** a bypass (6) is provided in order to at least intermittently bypass the heat exchanging means (3).

6. The apparatus according to claim 5, **characterized in that** a second control means is provided in order to control the bypass (6) as a function of the temperature of the gas containing dinitrogen monoxide, the temperature of the fixed bed reactor (5), the temperature of the waste gas, the extent of the waste gas return, the dinitrogen monoxide concentration and/or time.

7. The apparatus according to claim 1, **characterized in that** a diluent is provided in order to dilute the gas containing dinitrogen monoxide with another gas that is free of dinitrogen monoxide.

8. The apparatus according to any one of claims 1-7, **characterized in that** a plurality of adiabatic fixed bed reactors (5) are provided in series, wherein respective heat exchangers or coolers are optionally arranged between the individual fixed bed reactors (5).

9. The apparatus according to any one of the preceding claims, **characterized in that** the catalyst is suitable for decomposing dinitrogen monoxide at temperatures below 450°C, preferably below 400°C.

10. A process for decomposing dinitrogen monoxide, featuring:
a step of supplying a gas containing dinitrogen monoxide;
a heat exchanging step for exchanging heat between a waste gas and the gas containing dinitrogen monoxide;
a heating step for intermittently heating the gas containing dinitrogen monoxide;
a reaction step for catalytically decomposing the dinitrogen monoxide in the gas containing dinitrogen monoxide in a fixed bed reactor (5);
a step of discharging the waste gas from the fixed bed reactor (5) via the heat exchanger, wherein
the reaction step begins at a temperature below 800°C and takes place under adiabatic conditions, wherein
the reaction takes place at atmospheric pressure and the gas containing dinitrogen monoxide is conveyed over the fixed bed reactor (5) by means of a fan.

11. The process according to claim 10, **characterized in that** the gas containing dinitrogen monoxide is mixed with at least part of the waste gas upstream of the fixed bed reactor (5).

12. The process according to claim 11, **characterized in that** the mixing process takes place prior to the heating step.

13. The process according to any one of claims 10-12, **characterized in that** the heating step is controlled as a function of the temperature of the gas containing dinitrogen monoxide, the temperature of the fixed bed reactor (5), the temperature of the waste gas, the extent of the waste gas return, the dinitrogen monoxide concentration and/or time.

14. The process according to any one of claims 10-13, **characterized in that** the gas containing dinitrogen monoxide is entirely or partially conveyed past the heat exchanger step in a bypass (6) in an intermittent fashion.

15. The process according to claim 14, **characterized in that** the bypass (6) is controlled as a function of the temperature of the gas containing dinitrogen monoxide, the temperature of the fixed bed reactor (5), the temperature of the waste gas, the extent of the waste gas return, the dinitrogen monoxide concentration and/or time.

16. The process according to any one of claims 10-15, **characterized in that** the gas containing dinitrogen monoxide is diluted with another gas that is free of dinitrogen monoxide.

17. The process according to any one of claims 10-16, **characterized in that** the reaction step begins at a temperature below 450°C, preferably below 400°C.

## Revendications

1. Dispositif destiné à décomposer du gaz hilarant, avec :
une entrée de gaz (1) pour alimenter un gaz contenant du gaz hilarant ;
un premier moyen d'échange thermique (3), pour échanger de la chaleur entre un gaz d'échappement et le gaz contenant du gaz hilarant ;
un dispositif de chauffage (4) pour le chauffage temporaire du gaz contenant du gaz hilarant ;
un réacteur à lit fixe (5) dans lequel est logé un catalyseur pour décomposer le gaz dans le gaz contenant du gaz hilarant ;
une sortie de gaz à travers laquelle le gaz d'échappement quittant le réacteur à lit fixe peut être évacué par l'intermédiaire de l'échangeur thermique ;
le catalyseur étant adapté pour décomposer du gaz hilarant à des températures inférieures à 800°C ; et
le réacteur à lit fixe (5) étant conçu en tant que réacteur adiabatique,
**caractérisé en ce que**
le dispositif est conçu de telle sorte que la réaction pour la décomposition catalytique du gaz hilarant se déroule sous une pression atmosphérique et **en ce qu'**il est prévu une soufflante, pour diriger le gaz contenant du gaz hilarant par l'intermédiaire du réacteur à lit fixe (5).

2. Dispositif selon la revendication 1, **caractérisé en ce qu'**il est prévu un moyen de recyclage (7,8) pour mélanger au moins une partie du gaz d'échappement avec le gaz contenant du gaz hilarant, à l'avant du réacteur à lit fixe (5).

3. Dispositif selon la revendication 2, **caractérisé en ce que** le moyen de recyclage (7,8) est conçu pour mélanger une partie du gaz d'échappement avec le gaz contenant du gaz hilarant à l'avant du dispositif de chauffage (4).

4. Dispositif selon l'une quelconque des revendications 1 à 3, **caractérisé en ce qu'**il est prévu un premier moyen de commande pour commander le fonctionnement du dispositif de chauffage (4) en fonction de la température du gaz contenant du gaz hilarant, de la température du réacteur à lit fixe (5), de la température du gaz d'échappement, du volume de recyclage du gaz d'échappement, de la concentration en gaz hilarant et/ou du temps.

5. Dispositif selon l'une quelconque des revendications 1 à 4, **caractérisé en ce qu'**il est prévu une dérivation (6) pour contourner au moins temporairement le moyen d'échange thermique (3).

6. Dispositif selon la revendication 5, **caractérisé en ce qu'**il est prévu un deuxième moyen de commande, pour commander la dérivation (6) en fonction de la température du gaz contenant du gaz hilarant, de la température du réacteur à lit fixe (5), de la température du gaz d'échappement, du volume de recyclage du gaz d'échappement, de la concentration en gaz hilarant et/ou du temps.

7. Dispositif selon la revendication 1, **caractérisé en ce qu'**il est prévu un moyen de dilution pour diluer le gaz contenant du gaz hilarant avec un autre gaz exempt de gaz hilarant.

8. Dispositif selon l'une quelconque des revendications 1 à 7, **caractérisé en ce qu'**il est prévu une pluralité de réacteurs à lit fixe (5) adiabatiques en une rangée, en option, des échangeurs thermiques ou refroidisseurs respectifs étant prévus entre les réacteurs à lit fixe (5) individuels.

9. Dispositif selon l'une quelconque des revendications précédentes, le catalyseur étant adapté pour la décomposition de gaz hilarant à des températures inférieures à 450°C, de préférence inférieures à 400°C.

10. Procédé destiné à décomposer du gaz hilarant, avec :
une étape pour alimenter un gaz contenant du gaz hilarant ;
une étape d'échange thermique pour échanger de la chaleur entre un gaz d'échappement et le gaz contenant du gaz hilarant ;
une étape de chauffage pour le chauffage temporaire du gaz contenant du gaz hilarant ;
une étape de réaction pour la décomposition catalytique du gaz hilarant dans le gaz contenant du gaz hilarant dans un réacteur à lit fixe (5) ;
une étape pour évacuer le gaz d'échappement hors du réacteur à lit fixe (5), par l'intermédiaire de l'échangeur thermique ;
l'étape de réaction commençant à une température inférieure à 800°C et sous des conditions adiabatiques,
la réaction se déroulant sous une pression atmosphérique et le gaz contenant du gaz hilarant étant conduit au moyen d'une soufflante par l'intermédiaire du réacteur à lit fixe (5).

11. Procédé selon la revendication 10, **caractérisé en ce qu'**on mélange le gaz contenant du gaz hilarant avec au moins une partie du gaz d'échappement à l'avant du réacteur à lit fixe (5).

12. Procédé selon la revendication 11, **caractérisé en ce que** le mélange s'effectue avant l'étape de chauffage.

13. Procédé selon l'une quelconque des revendications 10 à 12, **caractérisé en ce que** l'étape de chauffage est commandée en fonction de la température du gaz contenant du gaz hilarant, de la température du réacteur à lit fixe (5), de la température du gaz d'échappement, du volume de recyclage du gaz d'échappement, de la concentration en gaz hilarant et/ou du temps.

14. Procédé selon l'une quelconque des revendications 10 à 13, **caractérisé en ce qu'**on fait passer temporairement, entièrement ou en partie le gaz contenant du gaz hilarant dans une dérivation (6), à l'avant de l'étape d'échangeur thermique.

15. Procédé selon la revendication 14, **caractérisé en ce que** la dérivation (6) est commandée en fonction de la température du gaz contenant du gaz hilarant, de la température du réacteur à lit fixe (5), de la température du gaz d'échappement, du volume de recyclage du gaz d'échappement, de la concentration en gaz hilarant et/ou du temps.

16. Procédé selon l'une quelconque des revendications 10 à 15, **caractérisé en ce qu'**on dilue le gaz contenant du gaz hilarant avec un autre gaz exempt de gaz hilarant.

17. Procédé selon l'une quelconque des revendications 10 à 16, **caractérisé en ce que** l'étape de réaction commence à une température inférieure à 450°C, de préférence inférieure à 400°C.
